# EUROPÄISCHE PATENTSCHRIFT

(11) **EP 1 241 979 B1**
(45) Veröffentlichungstag und Bekanntmachung des Hinweises auf die Patenterteilung: **28.01.2004**
(21) Anmeldenummer: 00979245.8
(22) Anmeldetag: 30.11.2000
(51) Int. Cl.: A61B 5/00, G01N 21/64

(54) **VERFAHREN UND VORRICHTUNG ZUR BESTIMMUNG DER ÖRTLICHEN VERTEILUNG EINER MESSGRÖSSE**
METHOD AND DEVICE FOR DETERMINING LOCAL DISTRIBUTION OF A MEASURING PARAMETER
PROCEDE ET DISPOSITIF PERMETTANT DE DETERMINER LA REPARTITION LOCALE D'UNE GRANDEUR DE MESURE

(30) Priorität: 14.12.1999 AT 210699
(43) Veröffentlichungstag der Anmeldung: 25.09.2002
(73) Patentinhaber: F. HOFFMANN-LA ROCHE AG, 4070 Basel (CH)
(72) Erfinder: HARTMANN, Paul, A-8160 Weiz (AT)
(74) Vertreter: Babeluk, Michael, Dipl.-Ing. Mag.
(86) Internationale Anmeldenummer: PCT/AT2000/000321
(87) Internationale Veröffentlichungsnummer: WO 2001/043628

(56) Entgegenhaltungen:
- EP-A- 0 354 204
- EP-A- 0 516 610
- GB-A- 2 033 575

## Beschreibung

Die Erfindung betrifft ein Verfahren zur Bestimmung der örtlichen Verteilung einer Messgröße bezogen auf einen oder in einem vorgegebenen, flächigen Messbereich einer biologischen Probe, vorzugsweise einer Organoberfläche oder der Epidermis, wobei in einem ersten Messverfahren zur Bestimmung einer ersten Messgröße ein Sensorfilm mit einem auf die Messgröße mit einer Änderung zumindest einer optischen Eigenschaft reagierenden Lumineszenzindikator auf den flächigen Messbereich aufgebracht und die erste Messgröße bildgebend detektiert wird, sowie eine Vorrichtung zur Durchführung des Verfahrens.

Für viele medizinische Bereiche, insbesondere der medizinischen Diagnostik, ist es von großer Bedeutung, die örtliche Verteilung einer Messgröße an einer Organoberfläche, beispielsweise der Haut, oder auch die Verteilung der Durchflussrate einer bestimmten Substanz durch eine Grenzfläche zu bestimmen. Weiters dienen derartige Messergebnisse auch der Überprüfung und Steuerung von medizinischen Therapieverfahren.

Beispielsweise ist die Sauerstoffversorgung der Haut eine wichtige Kenngröße für die Diagnose von Krankheiten, welche auf Störungen der Mikrozirkulation basieren. Die Sauerstoffversorgung wird einerseits von den Transporteigenschaften des Blutes (Perfusion) bestimmt, und andererseits von den transcutanen Transporteigenschaften und dem Verbrauch von Sauerstoff in der Haut. Zusätzlich wird die Sauerstoffversorgung zum Teil durch eine Sauerstoffaufnahme aus der Umgebung abgedeckt, was als Hautatmung bekannt ist. Schwierigkeiten bereitet die gleichzeitige Erfassung mehrerer Parameter, welche in unterschiedlichem Ausmaß die medizinisch relevante Größe beeinflussen.

Es ist bekannt, die Sauerstoffkonzentration oder den transcutanen Sauerstoffpartialdruck (tcPO₂) mit der Hilfe von Hautelektroden zu messen. Weiters wurden auch bereits optische Sensoren basierend auf der Fluoreszenzlöschung verwendet, welche den Vorteil aufweisen, im Gegensatz zu Elektroden bei der Messung keinen Sauerstoff zu verbrauchen.

Weiters ist es bekannt mit Hilfe von optischen Sensoren auf der Basis der Fluoreszenzlöschung ein sogenanntes Fluoreszenz-Lifetime-Imaging (FLIM) Verfahren anzuwenden, bei welchem eine Sensorfolie mit einem geeigneten Lumineszenzindikator auf den zu messenden Hautbereich fixiert wird. Aus der Hautoberfläche diffundierender Sauerstoff gelangt in die Sensorfolie und die daraus resultierende Fluoreszenzlöschung kann durch das Detektionssystem analysiert werden.

In diesem Zusammenhang ist aus der US 5,593.899 A eine Vorrichtung und ein Verfahren zur Messung der Sauerstoffversorgung der Haut bekannt geworden, bei welcher die von der Sauerstoffkonzentration abhängige Löschung eines Fluoreszenzindikators als Messgröße genützt wird. Zur Messung der Sauerstoffversorgung wird auf den entsprechenden Hautbereich eine lumineszierende Schicht als Bestandteil einer Hautcreme aufgetragen, und durch einen sauerstoffimpermeablen Film abgedeckt. Die optische Einrichtung zur Anregung des Indikators und Detektion der Messstrahlung ist in einem Gehäuse angeordnet, dessen transparente Abdeckung direkt auf den O₂-impermeablen Film aufgesetzt wird. Daran anschließend ist ein Interferenzfilter sowie eine Fotodiode angeordnet. Die lumineszierende Schicht wird über Lichtleiter von einer modulierten Strahlungsquelle mit Anregungsstrahlung beaufschlagt. Die beschriebene Anordnung eignet sich allerdings nur für eine integrale Messung über den gesamten, von der optischen Einrichtung erfassten Messbereich. Genaue Aussagen, beispielsweise über Grenzbereiche zwischen ausreichend mit Sauerstoff versorgten bzw. unterversorgten Gebieten sind mit dieser Einrichtung allerdings nicht möglich.

Aus der EP 0 516 610 B1 ist eine Vorrichtung bekannt geworden, mit welcher nicht nur die Sauerstoffkonzentration, sondern auch der Sauerstofffluss durch eine Grenzfläche, beispielsweise einem Hautbereich, gemessen werden kann. Die der Grenz- oder Messfläche zuordenbare Sensorschicht der Messvorrichtung setzt dem zu messenden Sauerstofffluss einen bekannten, endlichen Widerstand entgegen, wobei in der Sensorschicht zumindest ein optischer Indikator zur Bestimmung der Sauerstoffkonzentration auf einer Seite der Sensorschicht vorgesehen ist. Aus dem auf einer Seite der Sensorschicht gemessenen sowie auf der anderen Seite bekannten Konzentrationswert (aus der Umgebungsluft) wird der Materiefluss durch die Grenzfläche bestimmt. Die Sensorschicht kann gemäß einer Ausführungsvariante durch ein bildgebendes System (z.B. CCD) flächenförmig abgetastet werden, wodurch eine örtliche Verteilung des Sauerstoffflusses bzw. der Sauerstoffkonzentration gemessen werden kann.

Überlegungen und Messergebnisse über die örtliche Verteilung des Sauerstoffflusses bzw. der subcutanen Sauerstoffkonzentration und der Vorschlag für ein bildgebendes Messverfahren sind weiters in dem Artikel "Fluorescence Lifetime Imaging of the Skin PO₂: Instrumentation and Results" aus "Advances in Experimental Medicine and Biology, Vol. 428,P.605-611 (1997), Verlag Plenum Press N.Y. geoffenbart. In diesem Artikel wird eine Sensorfolie zur Messung der transcutanen Sauerstoffkonzentration beschrieben, welche eine der Hautoberfläche zugewandte optische Isolationsschicht, eine Sensorschicht mit einem Lumineszenzindikator mit O₂-sensitiver Abklingzeit und eine für Sauerstoff undurchlässige Trägerschicht aufweist. Eine ebenfalls beschriebene Membran zur Bestimmung des Sauerstoffflusses unterscheidet sich von der erstgenannten durch eine Diffusionsbarriere mit bekannter Sauerstoffdurchlässigkeit, welche anstelle der O₂impermeablen Schicht angeordnet ist. Zur Messung wird die Sensorfolie auf die Messoberfläche, beispielsweise einen Hautbereich, aufgebracht. Beim Messverfahren bedient man sich einer Modulationstechnik, wobei die die Anregungsstrahlung in Richtung der Sensormembran emittierenden LEDs durch einen Square-Wave-Generator angetrieben werden und eine rechteckförmig modulierte Anregungsstrahlung emittieren. Die von der Sensorfolie ausgehende Messstrahlung wird von einer CCD-Kamera mit modulierter Verstärkung erfasst und pixelweise an eine Rechnereinheit zur Bildverarbeitung weitergeleitet. Dokumentiert wird die in einer Polymerschicht gemessene Sauerstoffverteilung als Abbild der unterschiedlichen Sauerstoffverteilung eines Hautbereichs.

Weitere bildgebende Messverfahren unter Verwendung der Phasenfluorometrie sind aus der US 5 485 530 A bekannt geworden.

Ein vollständiges Bild über die Versorgung eines bestimmten Hautbereiches bzw. einer Organoberfläche, kann nur dann erhalten werden, wenn man neben dem Sauerstoffstatus für den betrachteten Bereich auch Informationen über die Blutversorgung bzw. die Perfusionsrate erhält.

Zur Gewinnung der notwendigen Perfusionsdaten stehen ausgereifte Verfahren und Vorrichtungen zur Verfügung, beispielsweise die Laser-Doppler-Flußmessung (siehe beispielsweise US 4 476 875 A), mit welcher die lokale Perfusion der Blutgefäße auf der Basis der Frequenzverschiebung einer von einer Laserlichtquelle emittierten Messstrahlung gemessen werden kann. Es ist auch möglich, die Messstrahlung durch geeignete optische Mittel aufzuweiten, oder aber Messbereiche in kleinen Schritten mit einem Laserstrahl abzutasten und so ein Bild über die örtliche Verteilung der Perfusionsrate zu gewinnen. Derartige Verfahren sind als Laser-Doppler-Imaging (LDI) Verfahren bekannt geworden. Das Ergebnis einer Laser-Doppler-Messung ist von der Geschwindigkeit und der Anzahl der roten Blutzellen, welche das Laserlicht streuen, abhängig.

So wird beispielsweise in der US 4 862 894 A eine Vorrichtung zur Untersuchung des Blutstromes in einem Hautbereich beschrieben, bei welcher ein durch eine Zylinderlinse verbreiterter Laserstrahl verwendet wird. In einer Ausführungsvariante wird die Hautoberfläche vom Laserstrahl zeilenförmig abgetastet und so ein zweidimensionales Bild der Verteilung der Strömungsgeschwindigkeit des Blutes erfasst. Weiters ist aus der US 5 361 769 A ein Verfahren und eine Vorrichtung zur Messung bewegter Flüssigkeiten bekannt geworden, wo durch Anwendung eines bildgebenden Laser-Doppler-Verfahrens ein Messobjekt flächig abgetastet wird. Über ein Linsensystem im Laserstrahl werden unterschiedliche Objektdistanzen ausgeglichen und so die Messgenauigkeit erhöht.

Die Gewinnung zufriedenstellender Messergebnisse über die Sauerstoffversorgung einer Organoberfläche oder eines Hautbereiches scheiterte bisher daran, dass sich die zu messenden Parameter rascher ändern als die dafür notwendigen, unterschiedlichen Messvorrichtungen bzw. Messverfahren im zu untersuchenden Hautbereich nacheinander angewandt werden können. Ein weiteres Problem besteht in der Heterogenität des Messbereiches, beispielsweise der Hautoberfläche, sodass punktförmige Messungen wie Elektroden- oder Laser-Doppler-Flußmessungen nicht zum Ziel führen und bildgebende Verfahren bzw. Vorrichtungen wie FLIM und LDI nur benachbart oder zeitlich nacheinander angewandt werden können. So verstreicht beispielsweise beim Wechsel von einer FLIM-Vorrichtung zu einer LDI-Vorrichtung für denselben Hautbereich ein zu großer Zeitraum im Hinblick auf die sich häufig in wenigen Sekunden ändernden Parameter (Perfusion und Sauerstoffstatus), sodass eine aussagekräftige Messung unmöglich wird. Weiters kann bei einem Wechsel der Vorrichtungen die exakte Repositionierung und somit die Erfassung der gleichen Messbereiche nur schwer garantiert werden.

Aufgabe der Erfindung ist es, ausgehend von den bekannten Messvorrichtungen und Verfahren ein Verfahren bzw. eine Vorrichtung zur Bestimmung der örtlichen Verteilung mehrerer Messgrößen in einem vorgegebenen, flächigen Messbereich einer biologischen Probe vorzuschlagen, welche den Anwender in die Lage versetzt, Aussagen über physiologische Größen und deren örtliche Zuordnung machen zu können.

Diese Aufgabe wird erfindungsgemäß dadurch gelöst, dass als erstes Messverfahren ein lumineszenzoptisches Verfahren verwendet wird, bei welchem die Lumineszenzabklingzeit oder eine davon abgeleitete Größe bezüglich der ersten Messgröße bildgebend erfasst wird und dass zur gleichzeitigen oder unmittelbar aufeinanderfolgenden Bestimmung der örtlichen Verteilung zumindest einer zweiten Messgröße bezogen auf den selben Messbereich ein durch den Sensorfilm wirkendes, zweites bildgebendes optisches Messverfahren verwendet wird.

Eine erfindungsgemäße Vorrichtung zur Durchführung des Verfahrens ist durch eine zweite, bildgebende optische Messeinrichtung gekennzeichnet, für deren Anregungs- und Messstrahlung der Sensorfilm der ersten optischen Messeinrichtung durchlässig ist, sodass zwei unabhängige optische Messeinrichtungen den selben Messbereich bildgebend erfassen. Die beiden Messungen können gleichzeitig oder so kurzfristig hintereinander durchgeführt werden, so dass die physiologischen Parameter in dieser kurzen Zeitspanne möglichst unverändert bleiben.

Ein wesentliches Merkmal der Erfindung besteht darin, die örtliche Verteilung von zumindest zwei Parametern oder Messgrößen einer biologischen Probe mit zwei voneinander unabhängigen, optischen Messverfahren bzw. Messvorrichtungen zu bestimmen, um die diagnostischen Aussagemöglichkeiten zu verbessern. Ein Messverfahren basiert auf der lumineszenzoptischen Bestimmung eines Parameters mit Hilfe einer einen Lumineszenzindikator enthaltenden Sensorfolie, für das zweite bzw. für weitere simultane, optische Messverfahren muss die Sensorfolie bzw. der Sensorfilm genügend transparent sein, um eine optische Messung mit ausreichender Signalgüte zuzulassen.

Als erstes Messverfahren wird ein lumineszenzoptisches Verfahren verwendet, bei welchem die Lumineszenzabklingzeit oder eine davon abgeleitete Größe bezüglich der ersten Messgröße bildgebend erfasst wird. Durch die Bestimmung der Abklingzeit ergibt sich der wesentliche Vorteil, dass die Messung unabhängig von der lokalen Beleuchtungsstärke des Sensorfilms ist. Damit können auch relativ stark gekrümmte Organ- bzw. Hautoberflächen mit einer einfachen Anordnung der Lichtquelle ausgeleuchtet werden, ohne auf homogene Anregungsbedingungen für den Lumineszenzindikator achten zu müssen. Es muss lediglich dafür gesorgt werden, dass in jedem Bereich der Sensorfolie ausreichend Lumineszenz für die Abklingzeitmessung zur Verfügung steht. Da die Abklingzeitmessung keiner Abhängigkeit von der Objektdistanz unterliegt, und die Laufzeit des Lichtes von unterschiedlich weit entfernten Objektoberflächen vernachlässigbar ist, kann die Abklingzeit auch an beliebig gekrümmten Messflächen ausreichend genau bestimmt werden. Damit ist eine wesentliche Voraussetzung erfüllt, um Messungen an Armen bzw. Beinen oder anderen gekrümmten Hautflächen von Patienten problemlos und sicher durchführen zu können. Bei der Erfassung des Lumineszenzsignals wird beispielsweise eine spezielle CCD-Kamera so betrieben, dass die Information in jedem Pixel mit der Abklingzeit am entsprechenden, dem Pixel zugeordneten Messort, in Beziehung gebracht werden kann. Alternativ kann anstelle einer CCD-Kamera auch ein CMOS-Sensor verwendet werden.

Als erste Messgröße kann beispielsweise die örtliche Verteilung der Sauerstoffkonzentration (tcPO₂), vorzugsweise die transcutane Sauerstoffkonzentration, oder die örtliche Verteilung des Sauerstoffflusses (O₂-Flux) durch die Organoberfläche, vorzugsweise die Hautoberfläche, gemessen werden.

Weiter ist es möglich als erste Messgröße die örtliche Verteilung der CO₂-Konzentration oder des CO₂-Flusses zu messen.

Bei Verwendung entsprechender Luminophore eignet sich das lumineszenzoptische Messverfahren zur Bestimmung der örtlichen Verteilung der Temperatur, der Glucosekonzentration oder einer Ionenkonzentration, beispielsweise des pH-Wertes.

Als zweites Messverfahren kann beispielsweise ein bildgebendes Laser-Doppler-Messverfahren (LDI-Verfahren) verwendet werden, mit welchem die Perfusionsrate im Messbereich bestimmt wird. Zur Erzielung einer hohen zeitlichen und örtlichen Korrelation für die Perfusionsrate und den Sauerstoffstatus einer biologischen Probe ist es notwendig, beide Messungen gleichzeitig oder beinahe gleichzeitig im selben Bereich der Probe vorzunehmen. Die hohe örtliche Auflösung moderner LDI-Vorrichtungen (< 100 µm) lässt sich ausgezeichnet mit einer Fluoreszenz-Lifetime-Imaging (FLIM) - Technik kombinieren, wodurch ausgezeichnete diagnostische Ergebnisse erzielt werden können.

Die Eigenschaften des Messverfahrens sollen - ohne die Erfindung darauf einzuschränken - anhand eines typischen Anwendungsbereiches, nämlich der Sauerstoffversorgung der menschlichen Haut, im folgenden näher dargelegt werden.

Ein wesentlicher Vorteil der Erfindung besteht darin, dass mit hoher zeitlicher und örtlicher Korrelation in einem ausgedehnten Untersuchungsbereich sowohl die lokale Sauerstoffkonzentration (erste Messgröße) im Sensorfilm (und damit auch in der interessierenden biologischen Probe), als auch die Perfusion im selben Untersuchungsbereich (zweite Messgröße) bestimmt, bildlich dargestellt und vergleichend beurteilt werden kann. Aus Kenntnis der lokalen Sauerstoffkonzentration in der Indikatorschicht des Sensorfilms kann je nach O₂-Diffusionseigenschaften der Trägerfolie und unter Berücksichtigung des barometrischen Luftdruckes der O₂-Partialdruck an der Hautoberfläche oder den O₂-Fluß errechnet werden. Durch die Messung der lokalen Perfusion im untersuchten Hautbereich kann auf die Durchblutung des Gewebes unter der Sensormembran geschlossen werden, wobei durch die hoch aufgelöste örtliche und zeitliche Korrelation zwischen Perfusion und tcPO₂ bzw. O₂-Flux ein umfassendes Bild der für die Hautversorgung entscheidenden Parameter (Perfusion und Sauerstoffgehalt) geliefert werden kann. Die Kombination dieser Parameter ermöglicht es dem Arzt, eine neue Qualität der Diagnose zu erreichen, die mit bisherigen Messverfahren aber auch mit jedem der kombinierten Messverfahren separat, nur beschränkt möglich war.

Als Ausführungsvariante der Erfindung kann als zweites oder weiteres Messverfahren ein photometrisches oder photographisches Verfahren verwendet werden, bei welchem der Messbereich in einem vorgegebenen Wellenlängenbereich bildgebend erfasst wird. Beispielsweise ist es möglich, auf diesem Wege die Autofluoreszenz oder die Infrarotstrahlung im Messbereich bildgebend zu erfassen. Mit Hilfe der Infrarotphotographie kann als Zusatzinformation beispielsweise die örtliche Wärmeverteilung eines Hautbereiches detektiert werden.

Während bei herkömmlichen lumineszenzoptischen Verfahren meist eine optisch isolierende Schicht zwischen der Haut und der Sensorfolie verwendet wird, um eine optische Entkopplung der Messstrahlung von der Hintergrundfluoreszenz der Probe zu erreichen, wird bei der vorliegenden Erfindung ein transparenter Sensorfilm verwendet, sodass andere Maßnahmen getroffen werden müssen, um Falschlichtanteile bei der Messung auszuschließen. Eine Möglichkeit besteht darin, Luminophore mit langer Abklingzeit (z.B. phosphoreszierende Porphyrine oder Übergangsmetallkomplexe) zu detektieren, wenn die zumeist kurzlebige Lumineszenz interferierender Substanzen (z.B. Melanin, Hämoglobin) der Probe bereits abgeklungen ist. Es können allerdings zur Signaltrennung auch verschiedene Phasenmesstechniken angewandt werden.

In einer weiteren Ausführungsvariante ist vorgesehen, dass als zweites oder weiteres Messverfahren ein profilometrisches, beispielsweise ein interferometrisches Messverfahren verwendet wird. Damit kann zusätzlich zum ersten Messwert die Topographie eines Organ- oder Hautbereiches in die Untersuchung einbezogen werden. Das Erfassen der dreidimensionalen Struktur des Messbereiches hat vor allem in der Dermatologie und der plastischen Chirurgie eine gewisse Bedeutung für die Diagnose und Therapie. Neben interferometrischen Methoden kann z. B. eine Streifenprojektionsmethode angewandt werden, bei welcher ein paralleles Strichgitter auf die zu vermessende Oberfläche projiziert und mit einer in einem bestimmten Winkel zur Projektionsebene angebrachten CCD-Kamera ausgewertet wird. Kleine Höhenunterschiede des betrachteten Profils erzeugen dabei eine Verzerrung des Streifenmusters, die quantitativ ausgewertet werden kann.

Weiters kann der Messbereich durch den Sensorfilm hindurch visuell begutachtet werden (beispielsweise auch durch Verfahren der Epilumineszenz-Mikroskopie). Erst durch die Verwendung eines transparenten Sensorfilms ergibt sich die Möglichkeit, den Messbereich visuell zu begutachten und morphologische Veränderungen, beispielsweise Farbänderungen oder Tumorbildungen direkt mit den Lumineszenzmesswerten des ersten Messverfahrens in Bezug zu setzen. Es ist auch möglich Markierungen an der Hautoberfläche anzubringen, welche durch die transparente Sensorfolie visuell oder fotographisch festgehalten werden können.

In einer Weiterbildung der Erfindung ist vorgesehen, dass durch die Sensorfolie verursachte Störungen der Messergebnisse des zweiten optischen Messverfahrens rechnerisch oder durch entsprechende Kalibrierparameter korrigiert werden. Der Messstrahl der LDI-Einheit erfährt beim Durchtritt durch den Sensorfilm eine geringe Abschwächung sowie eine Streuung. Wenn der Eintrittsvektor des LDI-Messstrahles nicht normal auf die Grenzfläche der Sensorfolie steht (bedingt durch die gekrümmte Oberfläche des Messobjektes), erfolgt auch eine geringfügige laterale Versetzung des Strahlverlaufs, die jedoch durch die geringe Dicke des Sensorfilms von etwa 50 µm vernachlässigt werden kann. Andererseits wird auch beim Durchtritt der Messstrahlung auf dem Weg von der Haut durch den Sensorfilm zum LDI-Detektor ein Anteil gestreut. Dadurch wird eine weitere Abweichung des Messwertes von jenem Wert, der ohne dazwischenliegender Sensorfolie gemessen würde, verursacht. Durch eine entsprechende rechnerische Korrektur bzw. eine Korrektur auf der Basis abgespeicherter Kalibrierwerte ist es jedoch möglich, den Einfluss der Sensorfolie weitestgehend zu eliminieren.

LDP-Messungen benötigen für einen kompletten Scan bzw. Abtastung der Messfläche oft einige Minuten, je nach geforderter Auflösung. Dies limitiert daher auch die zeitliche Auflösung des Gesamtsystems. FLIM-Messungen können in einem wesentlich kürzeren Zeitraum durchgeführt werden (Sekundenbruchteile). Um eine zeitlich ausreichend gute Korrelation zwischen LDI- und FLIM-Bildern des selben Messbereiches zu erzielen, kann beispielsweise vor und nach einer LDI-Messung jeweils eine FLIM-Messung durchgeführt werden.

Es ist jedoch auch möglich, die LDI- und FLIM-Messungen ineinander verschachtelt durchzuführen, indem man entweder den LDI-Scan kurzzeitig unterbricht um die nur Sekundenbruchteile dauernde FLIM-Messung durchzuführen oder die kurze Zeit der Repositionierung des Laserstrahls auf einen neuen Messpunkt für die FLIM-Messung nützt. Dabei können die Lichtquellen für die LDI- und die FLIM-Messung unter Umständen abwechselnd betrieben werden, da die Detektoren für die Messstrahlung des jeweils anderen Messverfahrens empfindlich sein können.

Prinzipiell ist es auch möglich, LDI- und FLIM-Messungen simultan durchzuführen. Dabei muss man jedoch gewährleisten, dass die detektierten Signale getrennt werden. Das geschieht entweder durch spektrale Unterscheidung zwischen LDI-Signal und Lumineszenzsignal und entsprechende Auswahl der Filter oder durch selektives elektronisches Filtern von unmodulierten und hochfrequent modulierten Signalen.

Die Erfindung wird im folgenden anhand von schematischen Zeichnungen näher erläutert.

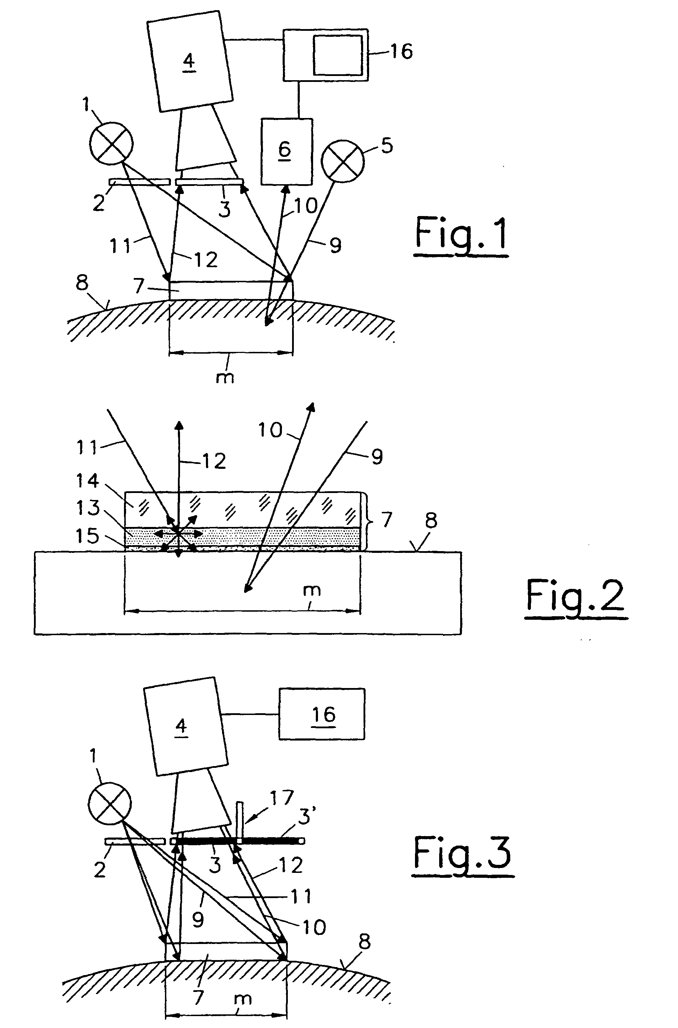

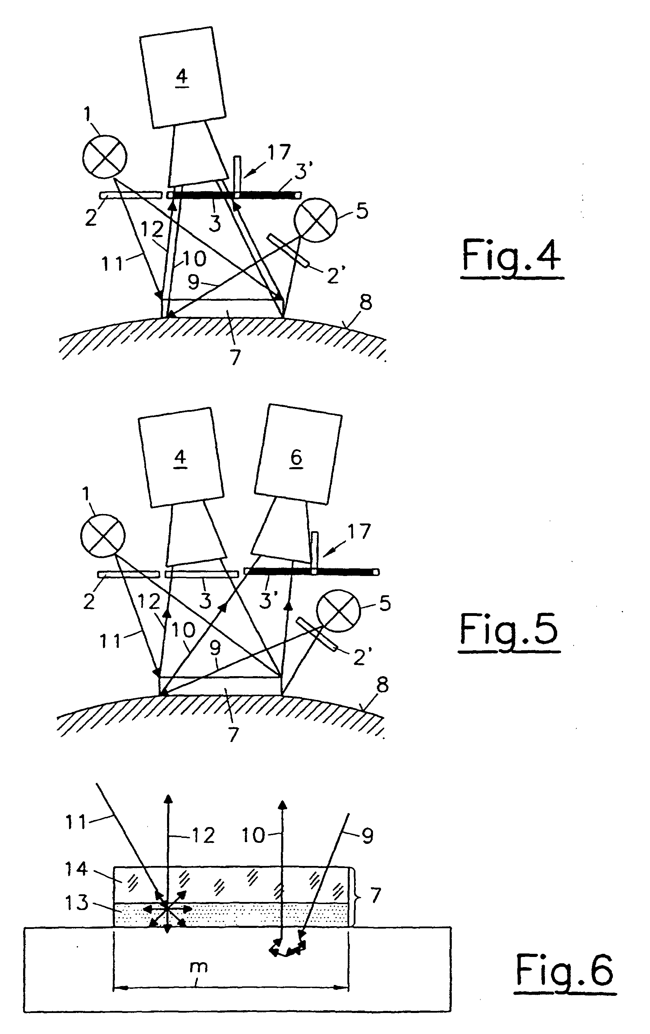

Es zeigen: Fig. 1 eine erste Variante der erfindungsgemäßen Vorrichtung, Fig. 2 ein Detail der Vorrichtung nach Fig. 1, Fig. 3 eine besonders einfache Ausführungsvariante, sowie die Fig. 4 bis 9 weitere vorteilhafte Ausführungsvarianten der Erfindung.

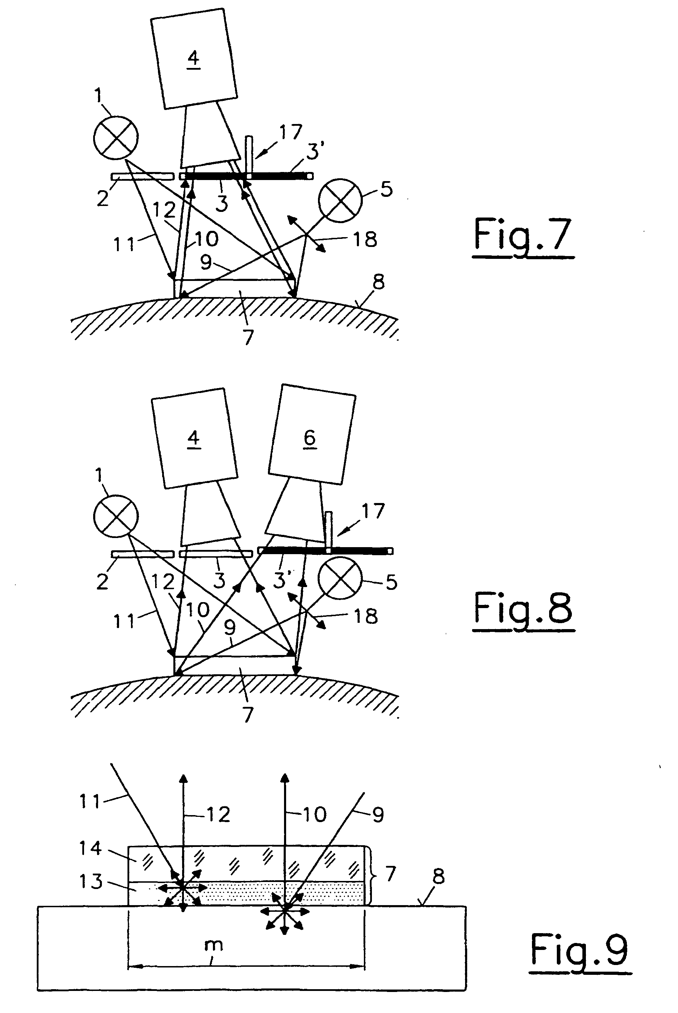

In den Figuren 1 und 2 ist schematisch eine erste Variante (Kombination FLIM mit LDI) der erfindungsgemäßen Vorrichtung beschrieben, mit welcher gleichzeitig für einen vorgegebenen Messbereich m die Sauerstoffkonzentration sowie die Perfusionsrate mit großer örtlicher Auflösung bestimmt werden kann. Das beispielhafte Messsystem besteht im wesentlichen aus einem Messwandler (Sensorfilm 7), einer Fluoreszenz-Lifetime-Imaging-Einheit (FLIM) und einer Laser-Doppler-Imaging-Einheit (LDI).
1. Sensorfilm: Der in Fig. 2 dargestellte Sensorfilm 7 weist eine transparente Trägerfolie 14 und auf der der Hautoberfläche 8 zugewandten Seite eine Indikatorschicht 13 auf. Die Indikatorschicht enthält einen Lumineszenzindikator, dessen Fluoreszenzlöschung in einem eindeutigen Zusammenhang mit der örtlich vorliegenden O₂-Konzentration steht. Die Indikatorschicht 13 muss die Anregungsstrahlung 11 der FLIM-Einheit in ausreichendem Maße absorbieren, wohingegen der gesamte Sensorfilm 7 inklusive Trägerfolie 14 für die Anregungs- und Messstrahlung 9, 10 der LDI-Einheit 5, 6 weitgehend transparent ist. Zwischen der Hautoberfläche 8 und der Indikatorschicht 13 kann eine Klebeschicht 15 vorgesehen sein.
   Eine typische Ausführungsvariante des Sensorfilms besteht aus einem flexiblen und transparenten polymeren Mehrschichtsystem, beginnend mit einer Trägerfolie 14, z.B. aus Polyester, Polyethylen, Zyklo-Olefin Co-polymerisat oder einem Fluorpolymer, und einer flexiblen und für die Messstrahlung des zusätzlichen Messverfahrens im wesentlichen transparenten Indikatorschicht 13. Letztere beinhaltet einen beispielsweise für Sauerstoff sensitiven Lumineszenz-Farbstoff, beispielsweise einen Ruthenium-diimin-Komplex, einen Osmium-diimin-Komplex, ein Platin-Porphyrin oder ein Palladium-Porphyrin, der in einer polymeren Matrix immobilisiert ist, beispielsweise in Silikon (mit oder ohne Kieselgel-Füllstoffen), Polyvinylchlorid (PVC) mit Weichmacher, Polymethylmethacrylat (PMMA) oder Polystyrol (PS).
   In einer weiteren Ausführungsform können auch Sensoren für den pH Wert oder den CO₂-Gehalt der Haut eingesetzt werden, die auf anderen Indikatoren, beispielsweise Hexa-pyren-trisulfonsäure, oder Naphthalimid basieren.
   Eine dritte polymere Schicht des Mehrschichtsystems, die den Sensor gegen die Epidermis begrenzt, ist vorzugsweise als Klebeschicht 15 ausgeführt, um einen durchgehenden Kontakt zur Hautoberfläche und eine einwandfreie Gasdiffusion zwischen Haut in den Sensor zu gewährleisten. Diese Klebeschicht besteht beispielsweise aus einem Gemisch aus Silikonharz und unvernetzten Silikonen ("Pressure-sensitive adhesive", PSA).
   In einer Ausführungsvariante (siehe Fig. 6 oder Fig. 9) kann auch die Matrix für die Immobilisierung des Messfarbstoffe selbst als Klebeschicht ausgeführt werden, indem man beispielsweise der Matrix ein PSA beimischt, oder das PSA selbst als Matrix für den Farbstoff verwendet.
2. FLIM-Einheit: Diese enthält alle jene mechanischen, elektronischen und optischen Komponenten die notwendig sind, um eine flächige Anregung der Indikatorschicht 13 und eine flächige, örtlich differenzierte Detektion der Messstrahlung 12 der Indikatorschicht 13 reproduzierbar durchzuführen. Als Anregungslichtquelle 1 dient beispielsweise eine blaue Leuchtdiöde, welche die Anregungsstrahlung 11 durch einen Anregungsfilter 2 auf den Sensorfilm 7 einstrahlt. Die Anregungsstrahlung 11 muss von der im wesentlichen transparenten Trägerfolie 14 durchgelassen, von der Indikatorschicht 13 des Sensorfilms 7 aber zumindest teilweise absorbiert werden. Die Lumineszenzstrahlung 12 aus der Indikatorschicht 13 wird über einen Emissionsfilter 3 von einer Detektionseinrichtung 4 vorzugsweise einer speziellen CCD-Kamera detektiert.
   Als Beispiel sind die spektroskopischen Daten für eine Sauerstoffmessung angeführt, bei welcher der Indikator (Ruthenium-diimin Komplexe) mit einer Lichtquelle (blaue LED) angeregt wird, deren Licht vom Indikator teilweise absorbiert wird: Das Absorptionsmaximum des Indikators liegt bei Wellenlängen zwischen 460 nm und 490 nm. Die Emission des Indikators hat ein Maximum zwischen 580 nm und 630 nm. Die maximale optische Dichte der Indikatorschicht im Wellenlängenbereich zwischen ca. 430 nm und 480 nm liegt dabei zwischen 0.05 und 0.5.
3. LDI-Einheit: Diese enthält alle jene mechanischen, elektronischen und optischen Komponenten, die notwendig sind, um den vom Sensorfilm 7 abgedeckten Messbereich m mit der Anregungsstrahlung 9 durch den Sensorfilm 7 hindurch abzutasten, die Messstrahlung 10 zu detektieren und zu analysieren, sowie gegebenenfalls eine Korrektur der Messwerte bezüglich der Streueigenschaften des Sensorfilms 7 vorzunehmen. Ein Laser 5 stellt monochromatische Strahlung einer Wellenlänge zur Verfügung, die vom Sensorfilm 7 nicht oder nur unwesentlich absorbiert wird. Dadurch bleibt die Anregungsstrahlung 9 für die Untersuchung der biologischen Probe, beispielsweise der Epidermis 8 beinahe ungeschwächt erhalten. Die Messstrahlung 10, die von der biologischen Probe 8 zurückgestreut bzw. reflektiert wird, enthält nun die Information, die vom Detektor 6 der LDI-Einheit empfangen und ausgewertet werden kann. Die FLIM- und die LDI-Einheit verfügen über eine gemeinsame Eingabe- und Auswerteeinheit 16.

In allen übrigen Ausführungsvarianten sind gleiche oder einander entsprechende Bauteile mit gleichen Bezugszeichen versehen.

In Fig. 3 ist eine sehr kompakte Ausführungsvariante dargestellt, bei welcher die zweite optische Messeinrichtung eine Einrichtung 17 zur Separierung der Messstrahlungen 10, 12 der beiden optischen Messeinrichtungen aufweist, beispielsweise ein Filterrad mit unterschiedlichen Emissionsfiltern 3, 3' für die beiden Messstrahlungen 10, 12. Die Vorrichtung weist lediglich eine Lichtquelle 1 sowie eine CCD-Kamera 4 auf. Diese Variante eignet sich besonders bei einer Kombination des FLIM-Verfahrens mit der Reflexions-Spektrophotometrie oder der Messung der Autofluoreszenz.

Die Fig. 4 bis 6 zeigen Vorrichtungen bei welchem das FLIM-Verfahren mit der Reflexions-Spektrophotometrie kombiniert wird. Dabei wird gemäß Fig. 4 eine breitbandige Lichtquelle als separate Anregungslichtquelle 5 verwendet, deren Anregungsstrahlung 9 über ein Anregungsfilter 2' auf die Probe 8 gerichtet wird. Für die Messung wird die CCD-Kamera 4 des FLIM-Verfahrens verwendet, welcher über die Emissionsfilter 3, 3' des Filterrades 17 die beiden Messstrahlungen 10, 12 zugeführt werden. Die Variante gemäß Fig. 5 unterscheidet sich von jener nach Fig. 4 durch die Verwendung einer separaten bildgebenden Detektionseinrichtung 6 (beispielsweise eine zweite CCD-Kamera), welcher die Messstrahlung 10 der Reflexions-Spektrophotometrie zugeführt wird. Fig. 6 zeigt die Wechselwirkung der Anregungsstrahlung 11 (FLIM) mit der Indikatorschicht 13 sowie die Streuung der Anregungsstrahlung 9 in der Epidermis 8.

Die Fig. 7 bis 9 zeigen Vorrichtungen bei welchem das FLIM-Verfahren mit der Messung der Autofluoreszenz kombiniert wird. Dabei wird gemäß Fig. 7 ein Laser als separate Anregungslichtquelle 5 verwendet, deren Licht durch einen Strahlaufweiter (Beam Expander) 18 auf die Probe 8 gerichtet wird. Die Messstrahlung 10 kann entweder über ein Filterrad 17 von der CCD-Kamera 4 (Fig. 7) oder von einer separaten bildgebenden Detektionseinrichtung 6 (Fig.8) erfasst werden. Fig. 9 zeigt die Wechselwirkung der Anregungsstrahlung 11 (FLIM) mit der Indikatorschicht 13 sowie jene der Anregungsstrahlung 9 (Autofluoreszenz) in der Epidermis 8.

## Patentansprüche

1. Verfahren zur Bestimmung der örtlichen Verteilung einer Messgröße bezogen auf einen oder in einem vorgegebenen, flächigen Messbereich einer biologischen Probe, vorzugsweise einer Organoberfläche oder der Epidermis, wobei in einem ersten Messverfahren zur Bestimmung einer ersten Messgröße ein Sensorfilm mit einem auf die Messgröße mit einer Änderung zumindest einer optischen Eigenschaft reagierenden Lumineszenzindikator auf den flächigen Messbereich aufgebracht und die erste Messgröße bildgebend detektiert wird, **dadurch gekennzeichnet, dass** als erstes Messverfahren ein lumineszenzoptisches Verfahren verwendet wird, bei welchem die Lumineszenzabklingzeit oder eine davon abgeleitete Größe bezüglich der ersten Messgröße bildgebend erfasst wird und dass zur gleichzeitigen oder unmittelbar aufeinanderfolgenden Bestimmung der örtlichen Verteilung zumindest einer zweiten Messgröße bezogen auf den selben Messbereich ein durch den Sensorfilm wirkendes, zweites bildgebendes optisches Messverfahren verwendet wird.

2. Verfahren nach Anspruch 1, **dadurch gekennzeichnet, dass** als erste Messgröße die örtliche Verteilung der Sauerstoffkonzentration (tcPO₂), vorzugsweise die transkutane Sauerstoffkonzentration, oder die örtliche Verteilung des Sauerstoffflusses (O₂-Flux) durch die Organoberfläche, vorzugsweise die Hautoberfläche, gemessen wird.

3. Verfahren nach Anspruch 1, **dadurch gekennzeichnet, dass** als erste Messgröße die örtliche Verteilung der CO₂-Konzentration oder die örtliche Verteilung des CO₂-Flusses gemessen wird.

4. Verfahren nach Anspruch 1, **dadurch gekennzeichnet, dass** als erste Messgröße die örtliche Verteilung der Temperatur, der Glucosekonzentration oder einer Ionenkonzentration, beispielsweise des pH-Wertes, gemessen wird.

5. Verfahren nach einem der Ansprüche 1 bis 4, **dadurch gekennzeichnet, dass** als zweites Messverfahren ein bildgebendes Laser-Doppler-Messverfahren verwendet wird, mit welchem die Perfusionsrate im Messbereich bestimmt wird.

6. Verfahren nach einem der Ansprüche 1 bis 5, **dadurch gekennzeichnet, dass** als zweites oder weiteres Messverfahren ein photometrisches oder photografisches Verfahren verwendet wird, bei welchem der Messbereich in einem vorgegebenen Wellenlängenbereich bildgebend erfasst wird.

7. Verfahren nach Anspruch 6, **dadurch gekennzeichnet, dass** die Autofluoreszenz im Messbereich bildgebend erfasst wird.

8. Verfahren nach Anspruch 6, **dadurch gekennzeichnet, dass** die Infrarotstrahlung im Messbereich bildgebend erfasst wird.

9. Verfahren nach einem der Ansprüche 1 bis 8, **dadurch gekennzeichnet, dass** als zweites oder weiteres Messverfahren ein profilometrisches , beispielsweise ein interferometrisches Messverfahren verwendet wird.

10. Verfahren nach einem der Ansprüche 1 bis 9, **dadurch gekennzeichnet, dass** als weiteres Messverfahren der Messbereich durch den Sensorfilm hindurch visuell begutachtet wird.

11. Verfahren nach einem der Ansprüche 1 bis 9, **dadurch gekennzeichnet, dass** durch die Sensorfolie verursachte Störungen der Messergebnisse des zweiten optischen Messverfahrens rechnerisch oder durch entsprechende Kalibrierparameter korrigiert werden.

12. Vorrichtung zur Bestimmung der örtlichen Verteilung einer Messgröße in einem vorgegebenen, flächigen Messbereich (m) einer biologischen Probe (8), vorzugsweise einer Organoberfläche oder der Epidermis mit einer ersten Messeinrichtung mit einer Anregungseinheit (1, 2) zur Bereitstellung einer Anregungsstrahlung (11), mit einer bildgebenden Detektionseinrichtung (3, 4) samt Auswerteeinrichtung (16), mit einem im Messbereich (m) aufbringbaren, flächigen Sensorfilm (7) mit bekannten Diffusionseigenschaften, für die zu bestimmende Messgröße, wobei der Sensorfilm (7) einen auf die zu bestimmende Messgröße mit einer Änderung zumindest einer optischen Eigenschaft reagierenden Lumineszenzindikator aufweist, **dadurch gekennzeichnet, dass** eine zweite, bildgebende optische Messeinrichtung (5; 6; 17) vorgesehen ist, für deren Anregungs- und Messstrahlung (9, 10) der Sensorfilm (7) der ersten optischen Messeinrichtung durchlässig ist, sodass zwei unabhängige optische Messeinrichtungen den selben Messbereich (m) bildgebend erfassen.

13. Vorrichtung nach Anspruch 12, **dadurch gekennzeichnet, dass** die zweite optische Messeinrichtung eine Einrichtung (17) zur Separierung der Messstrahlungen (10, 12) der beiden optischen Messeinrichtungen aufweist, beispielsweise ein Filterrad mit unterschiedlichen Emissionsfiltem (3, 3') für die beiden Messstrahlungen (10, 12).

14. Vorrichtung nach Anspruch 12 oder 13, **dadurch gekennzeichnet, dass** die zweite optische Messeinrichtung eine separate Anregungslichtquelle (5) aufweist.

15. Vorrichtung nach Anspruch 12, **dadurch gekennzeichnet, dass** die zweite optische Messeinrichtung eine separate Anregungslichtquelle (5) sowie eine separate bildgebende Detektionseinrichtung (6) aufweist.

## Claims

1. Method for determining the local distribution of a quantity to be measured relative to or present in a predefined measurement area of a biological sample, preferably the surface of an organ or the epidermis, where in a first measuring process for determining a first measurement variable a sensor film with a luminescence indicator reacting to this variable by a change of at least one optical characteristic, is applied to the measurement area and the first measurement variable is detected by imaging means, **characterized in that** a luminescence-optical method is used as first measuring process, in which luminescence decay time or a quantity derived therefrom as a function of a first measurement variable is recorded by an imaging technique, and that for simultaneous or immediately following determination of the local distribution of at least one further variable in the same measurement area a second optical measuring process using an imaging technique is employed which is effective through the sensor film.

2. Method according to claim 1, **characterized in that** as first quantity to be measured local distribution of oxygen concentration (tcPO₂), and preferably transcutaneous oxygen concentration, or local distribution of oxygen flow (O₂-flux) through the organ surface, preferably the skin surface, will be determined.

3. Method according to claim 1, **characterized in that** as first quantity to be measured local distribution of CO₂ concentration or CO₂ flow will be determined.

4. Method according to claim 1, **characterized in that** as first quantity to be measured local distribution of temperature, glucose concentration, or an ionic concentration, such as pH level will be determined.

5. Method according to any of claims 1 to 4, **characterized in that** a Laser Doppler imaging process is used as second measuring process, by means of which the perfusion rate in the measurement area is determined.

6. Method according to any of claims 1 to 5, **characterized in that** a photometric or photographic method is used as second or further measuring process, where the measurement area is recorded as an image in a predefined range of wavelengths.

7. Method according to claim 6, **characterized in that** autofluorescence in the measurement area is recorded as an image.

8. Method according to claim 6, **characterized in that** infrared radiation in the measurement area is recorded as an image.

9. Method according to any of claims 1 to 8, **characterized in that** a profilometric method, e.g., an interferometric method is employed as second or further measuring process.

10. Method according to any of claims 1 to 9, **characterized in that** the measurement area is assessed by visual inspection through the sensor film as further measuring process.

11. Method according to any of claims 1 to 9, **characterized in that** any errors in the measured results of the second optical measuring process, which are due to the sensor film, are corrected by a computing procedure or suitable calibrating parameters.

12. System for determining the local distribution of a quantity to be measured in a predefined measurement area (m) of a biological sample (8), preferably the surface of an organ or the epidermis, comprising a first measuring device with an excitation unit (1, 2) for supply of excitation radiation (11), and an image-producing detection unit (3, 4) including an evaluation unit (16), and a sensor film (7) to be applied to the measurement area (m), whose diffusion properties concerning the variable to be measured are known, the sensor film (7) featuring a luminescence indicator reacting to said measurement variable by a change of at least one optical characteristic, **characterized in that** a second image-producing optical measuring device (5; 6; 17) is provided for whose excitation and emission radiation (9, 10) the sensor film (7) of the first optical measuring device is transparent, so that two independent optical measuring means will cover the same measurement area (m).

13. System according to claim 12, **characterized in that** the second optical measuring device is provided with a unit (17) for separation of radiations (10, 12) emitted by the two optical measuring devices, such as a filter wheel with different emission filters (3, 3') for the two radiations (10, 12).

14. System according to claim 12 or 13, **characterized in that** the second optical measuring device is provided with a separate excitation lightsource (5).

15. System according to claim 12, **characterized in that** the second optical measuring device is provided with a separate excitation lightsource (5) and a separate image-producing detection unit (6).

## Revendications

1. Procédé de détermination de la distribution locale d'une grandeur de mesure en se référant sur une ou dans une zone dé mesure plate, prédéterminée, d'un échantillon biologique, de préférence d'une surface organique ou d'un épiderme, sachant que, dans un premier procédé de mesure de détermination d'une première grandeur de mesure, un film capteur, ayant un indicateur de luminescence réagissant à la grandeur de mesure par une modification d'au moins une propriété optique, est appliqué sur la zone de mesure plate, et la première grandeur de mesure est détectée, avec formation d'une image, **caractérisé en ce que** l'on utilise comme premier procédé de mesure un procédé optique à luminescence, dans lequel le temps d'évanouissement de la luminescence, ou bien une grandeur en étant dérivée, par rapport à la première grandeur de mesure, est détectée, avec formation d'image, et **en ce que**, pour déterminer simultanément ou bien directement subséquemment la distribution locale d'au moins une deuxième grandeur de mesure se référant à la même zone de mesure, on utilise un deuxième procédé de mesure optique, avec formation d'image, agissant au moyen du film capteur.

2. Procédé selon la revendication 1, **caractérisé en ce que** l'on mesure comme première grandeur de mesure la distribution locale de la concentration en oxygène (tcPO₂), de préférence la concentration en oxygène transcutanée, ou bien la distribution locale du flux d'oxygène (O₂-flux) à travers la surface organique, de préférence la surface de la peau.

3. Procédé selon la revendication 1, **caractérisé en ce qu'**on mesure comme première grandeur de mesure la distribution locale de la concentration de CO₂, ou bien la distribution locale du flux de CO₂.

4. Procédé selon la revendication 1, **caractérisé en ce qu'**on mesure, à titre de première grandeur de mesure, la distribution locale de la température de la concentration en glucose ou d'une concentration d'ions, par exemple de la valeur du pH.

5. Procédé selon l'une des revendications 1 à 4, **caractérisé en ce qu'**on utilise, à titre de deuxième procédé de mesure, un procédé à laser et effet Doppler, formateur d'image, à l'aide duquel on détermine le taux de perfusion dans la zone de mesure.

6. Procédé selon l'une des revendications 1 à 5, **caractérisé en ce qu'**on utilise comme deuxième ou comme autre procédé de mesure un procédé photométrique ou photographique, pour lequel la zone de mesure est détectée, avec formation d'image, dans une plage de longueurs d'onde prédéterminée.

7. Procédé selon la revendication 6, **caractérisé en ce que** l'autofluorescence est détectée, avec formation d'image, dans la zone de mesure.

8. Procédé selon la revendication 6, **caractérisé en ce que** le rayonnement infrarouge est détecté, avec formation d'image, dans la zone de mesure.

9. Procédé selon l'une des revendications 1 à 8, **caractérisé en ce qu'**on utilise comme deuxième ou autre procédé de mesure un procédé de mesure profilométrique, par exemple un procédé de mesure interférométrique.

10. Procédé selon l'une des revendications 1 à 9, **caractérisé en ce qu'**on expertise à titre d'autre procédé de mesure la zone de mesure, visuellement, au moyen d'un film capteur.

11. Procédé selon l'une des revendications 1 à 9, **caractérisé en ce que** les perturbations des résultats de mesure, provoquées par la feuille capteur du deuxième procédé de mesure optique, sont corrigées par calcul ou par des paramètres d'étalonnage correspondants.

12. Dispositif de détermination de la distribution locale d'une grandeur de mesure dans une zone de mesure (m) plate, prédéterminée, d'une sonde biologique (8), de préférence une surface organique ou l'épiderme, avec un premier dispositif de mesure muni d'une unité excitatrice (1,2) pour fournir un rayonnement d'excitation (11), avec un dispositif de détection (3,4) formateur d'image, muni d'un dispositif d'évaluation (16), avec un film de capteur (7) plat, pouvant être appliqué dans la zone de mesure (m) et présentant des propriétés de diffusion connues, pour la grandeur de mesure à déterminer, sachant que le film de capteur (7) présente un indicateur de luminescence qui réagit à la grandeur de mesure à déterminer, par une modification d'au moins une propriété optique, **caractérisé en ce qu'**est prévu un deuxième dispositif de mesure optique (5; 6; 17) formateur d'image, pour le rayonnement d'excitation et de mesure (9,10) duquel le film de capteur (7) du premier dispositif de mesure optique est transparent, de sorte que deux dispositifs de mesure optiques indépendants détectent avec formation d'image la même zone de mesure (m).

13. Dispositif selon la revendication 12, **caractérisé en ce que** le deuxième dispositif de mesure optique présente un dispositif (17) de séparation des rayons de mesure (10,12) des deux dispositifs de mesure optiques, par exemple une roue filtrante, munie de filtres à émission (3,3') différents, pour les deux rayonnements de mesure (10,12).

14. Dispositif selon la revendication 12 ou 13, **caractérisé en ce que** le deuxième dispositif de mesure optique présente une source de lumière excitatrice (5) séparée.

15. Dispositif selon la revendication 12, **caractérisé en ce que** le deuxième dispositif de mesure optique présente une source de lumière excitatrice (5) séparée, ainsi qu'un dispositif de détection (6) formateur d'image, séparé.
